# EUROPEAN PATENT SPECIFICATION

(11) **EP 2 104 483 B1**
(45) Date of publication and mention of the grant of the patent: **20.07.2016**
(21) Application number: 08701007.0
(22) Date of filing: 08.01.2008
(51) Int. Cl.: A61K 8/04, A61K 8/06, A61K 8/37, A61K 8/39, A61K 8/46, A61K 8/55, A61K 9/107, A61K 9/12, A61K 9/127, A61K 9/51, A61Q 19/00

(54) **COLLOIDAL SOLUTION**
KOLLOIDALE LÖSUNG
SOLUTION COLLOÏDALE

(30) Priority: 16.01.2007 EP 07290053
(43) Date of publication of application: 30.09.2009
(73) Proprietor: Bayer Consumer Care AG, 4052 Basel (CH)
(72) Inventor: DEPERRAZ, Françoise, F-74100 Ville-la-Grand (FR); BAROTH, Volker, CH-4102 Binningen (CH)
(74) Representative: BIP Patents
(86) International application number: PCT/EP2008/000064
(87) International publication number: WO 2008/086953

(56) References cited:
- EP-A- 0 852 941
- EP-A- 0 956 851
- EP-A- 0 956 853
- WO-A-96/37192
- WO-A-97/21428
- US-A1- 2004 001 871

## Description

The present invention relates to a composition which is a colloidal solution comprising at least one active ingredient, at least one membrane-forming molecule and at least one foaming component, its process for preparation and its cosmetically or pharmaceutical use.

Pharmaceutical and cosmetic compositions for skin care exist in various forms of preparation e.g. as gels, lotions, foams, sprays, ointments, pastes, creams, etc.. Due to their adhesion to the epidermis and their penetration properties, such compositions are helpful for wound healing, compensation for loss of moisture or fat form the skin, and regeneration and protection of the skin in connection with environmental influences, weather, UV radiation from sunlight etc..

In order to reach the desired effect the active ingredient has to be transported to the respective site, for example to the skin surface, mucosa, nails, hair, epidermal and dermal areas of the skin by means of carrier or transport vehicles. Therefore the active ingredients must be sufficiently soluble in the formulation base or at least adequately solubilized and must be compatible with the other excipients of the formulation. In many cosmetics the active ingredients, for example water soluble vitamins or melanines, or amino acids, are encapsulated in liposomes. The composition comprising the active ingredient, the formulation base and other excipients should be homogenous, must be adequately stable and must not tend to e.g. chemical reactions or separation effects. In particular nanodispersions, a dispersion of nanoparticles, on a liposome basis shows advantageous properties in this regard.

EP 0 852 941, EP 0 956 851 and EP 0 956 853 describe stable nanodispersions comprising an oil-soluble active ingredient e.g. vitamin A, vitamin E or dexpanthenol, a specific phospholipid and a partial fatty acid ester of polyoxyethylene sorbitan as an emulsifier. Especially EP 0852 941 mentions Polysorbatum 80 as said partial fatty acid ester in the disclosed examples.

### Field of invention

The present invention relates to a colloidal solution as basis for a foam spray comprising at least one active ingredient, at least one membrane-forming molecule, and at least one foaming component, whereby the active ingredient is dexpanthenol, the membrane-forming molecule is is phosphatidylcholine, and the foaming component is selected from the group consisting of sodium oleate, sodium cocoamphoacetate, capryl/capramidopropyl betaine, a mixture of disodium PEG-5 laurylcitrate / sulfosuccinat / sodium laureth sulphate, a mixture of sodium lauryl glucose carboxylate / lauryl glucoside, or a mixture thereof.

A colloidal solution according to the invention is an especially finely dispersed system of a lipophilic phase in an aqueous or aqueous-alcoholic phase based on nanoparticles having a particle size less equal than 100 nm.

A colloidal solution according to the invention has opalescence and transparency in back lighting, which hints that the colloidal solution is physically identical to the ideal state of a true molecular solution. Electron-microscopic images show a homogenous dispersion of particles with a particle size of less than about 100 nm, so called nanoparticles. To characterize the available colloidal solution, methods known in the art are suitable, e.g., optical evaluation: weak to strong opalescence, dynamic light scattering (e.g. Photon Correlation Spectroscopy) and electron microscopy. Laser light scattering measurements and electron microscopic analysis confirm the very small size and excellent homogeneity of the nanoparticles present in the composition. Such colloidal solution is sometimes called as nanodispersion.

The colloidal solution according to the invention shows surprisingly a long shelf life, i.e. no separation and high stability of the particle size after stowage for several weeks at temperatures up to 50°C.

According to an embodiment the active ingredients can be a water-soluble and/or an oil-soluble compound preferably suitable for skin preparations.

Examples of water-soluble ingredients include but are not limited to dexpanthenol, pantothenic acid, folic acid, vitamin C, enzymes, extracts of planets, or mixtures thereof. Preference is given to dexpanthenol.

Further examples for active ingredients include but are not limited to oil-soluble vitamins, allantoin, azulene, cosmetic and therapeutic oils, oil-soluble sunblocks or mixtures thereof.

Examples of oil-soluble ingredients include but are not limited to vitamin A, retinol in the form of free acid or its derivatives, vitamin E, essential fatty acids, or mixtures thereof.

Example of sunblocks include but are not limited to anthranilic acid derivatives, salicylic acid derivatives, cinnamic acid ester derivatives, coumarin derivatives, o-aminobenzoic acid derivatives, benzophenone, benzylidene camphor or mixtures thereof. Preference is given to 4-aminobenzoic acid, 2-ethylhexyl-4-methoxy-cinnamate, 2-ethylhexylsalicylate, benzophenone-4, benzophenone-3, benzophenone-10 and mixtures thereof. Furthermore suitable sunblocks are described in Pflegekosmetik: Ein Leitfaden [Cosmetics: A Manual], W. Raab, U. Kindl, Govi-Verlag, Frankfurt 1991 and in Ullmann's Encyclopaedia of Industrial Chemistry, VCH, 5th edition, Vol. A24, entry skin cosmetics.

The active ingredient is present in the colloidal solution according to the invention in a concentration of e.g. from 0.1 up to 20%, preferably from 1 up to 10% by weight, based on the total weight of the colloidal solution. Preference is given to dexpanthenol as active ingredient in a concentration of preferably from 1 up to 10 %, more preferably from 1.5 up to 7 % by weight based on the total weight of the colloidal solution.

Further examples of membrane-forming molecules include but are not limited to phospholipids such as lecithin which can be a mixture of different phospholipids, hydrogenated or partially hydrogenated phospholipids, lysophospholipids, ceramide or mixtures thereof. Preference is given to lecithin as a membrane-forming molecule. Most preferably the phospholipid phosphatidylcholine is used as membrane-forming molecule.

The phosphatidylcholine or lecithin can stem for example from egg yolk or soy beans. The phosphatidylcholine stemmed from egg yolk contains for example C16-fatty acid (16:0) in an amount of around 33%, fatty acid (18:0) in an amount of around 14%, fatty acid (18:1) in an amount of around 30%, fatty acid (18:2) in an amount of around 14% and fatty acid (20:4) in an amount of around 4% by weight. The phosphatidylcholine stemmed from soy beans contains for example fatty acid (16:0) in an amount of around 14%, fatty acid (18:0) in an amount of around 4%, fatty acid (18:1) in an amount of around 12%, fatty acid (18:2) in an amount of around 65% and fatty acid (18:3) in an amount of around 5% by weight. The phosphatidylcholine stemmed from soy beans is preferred.

The membrane-forming molecule is present in the colloidal solution according to the invention in a concentration of e.g. from 0.05 up to 15%, preferably from 0.1 up to 5%, more preferably from 0.2 up to 1% by weight based on the total weight of the colloidal solution. Preference is given to phosphatidylcholine as active ingredient in a concentration of preferably from 0.1 up to 2 %, more preferably from 0.2 up to 0.8% by weight based on the total weight of the colloidal solution.

The foaming component according to the invention is selected from the group consisting of sodium oleate, sodium cocoamphoacetate, capryl/capramidopropyl betaine, a mixture of disodium PEG-5 laurylcitrate / sulfosuccinat / sodium laureth sulphate, a mixture of sodium lauryl glucose carboxylate / lauryl glucoside, or a mixture thereof. More preferably cocoamphoacetate, capryl/capramidopropyl betaine, a mixture of lauryl glucose carboxylate / lauryl glucoside or a mixture thereof are used as foaming component. Most preferably sodium cocoamphoacetate, capryl/capramidopropyl betaine, the mixture of sodium lauryl glucose carboxylate / lauryl glucoside or a mixture thereof are used as foaming component. The foaming agents according to the invention can also be understood as surfactants.

The foaming component is present in the colloidal solution according to the invention in a concentration of e.g. from 0.5 up to 10%, preferably from 1 up to 4% by weight based on the total weight of the colloidal solution.

In addition the colloidal solution according to the invention can comprise sodium oleate as surfactant e.g. in a concentration of from 0.01 up to 0.1%, preferably from 0.01 up to 0.05% by weight based on the total weight of the colloidal solution.

Surprisingly the colloidal solution according to the invention remains stable in the presence of the foaming agent.

Furthermore the colloidal solution according to the invention can include one or more solvent, solubilizer, buffering agent, chelating agent, cooling agent and/or preservative.

Examples of solvents include but are not limited to water, preferably purified water, C₂-C₈ alcohol, preferably ethanol or propylene glycol, or mixtures thereof. Preference is given to water as solvent.

The solvent can be present in the colloidal solution according to the invention in a concentration of e.g. from 50 up to 95%, preferably from 70 up to 92% by weight based on the total weight of the colloidal solution. Preference is given to water in a concentration of from 80 up to 95% based on the total weight of the colloidal solution.

Examples of solubilizers include but are not limited to C₂-C₈ alcohol, preferably ethanol or propylene glycol, or mixtures thereof. Preference is given to ethanol as solubilizer.

The solubilizer can be present in the colloidal solution according to the invention in a concentration of e.g from 0.1 up to 2%, preferably from 0.2 up to 0.8 %, more preferably from 0.35 up to 0.55% by weight based on the total weight of the colloidal solution.

Examples of buffering agents include but are not limited to sodium dihydrogen phosphate, disodium phosphate, or a mixture thereof. Preference is given to sodium dihydrogen phosphate, disodium phosphate or a mixture thereof.

The buffering agent can be present in the colloidal solution according to the invention in a concentration of e.g. from 0.01 up to 1% by weight based on the total weight of the colloidal solution.

Sodium dihydrogen phosphate as buffering agent can be used in a concentration of e.g. from 0.05 up to 0.2%, preferably from 0.1 up to 0.15% by weight based on the total weight of the colloidal solution.

Disodium phosphate as buffering agent can be used in a concentration of e.g. from 0.01 up to 0.1%, preferably from 0.01 up to 0.05% by weight based on the total weight of the colloidal solution.

Preference is given to a mixture of sodium dihydrogen phosphate and disodium phosphate in a ratio of e.g. 10:1 up to 1:1, preferably 7:1 up to 3:1 in the colloidal solution according to the invention.

Examples of chelating agents include but are not limited to sodium edetate. Preference is given to sodium edetate.

The chelating agent can be present in the colloidal solution according to the invention in a concentration of e.g. from 0.01 up to 1%, preferably from 0.02 up to 0.08% by weight based on the total weight of the colloidal solution.

Examples of preservatives include but are not limited to phenoxyethanol. Preference is given to Phenoxyethanol.

The preservative can be present in the colloidal solution according to the invention in a concentration of e.g. from 0.1 up to 1 % by weight based on the total weight of the colloidal solution.

Examples of cooling agents include but are not limited to isobutane and isopentane, or a mixture thereof. Preference is given to a mixture of isobutane and isopentane.

The cooling agent can be present in the colloidal solution according to the invention in a concentration of e.g. from 0.1 up to 10%, preferably from 0.5 up to 5%, more preferably from 1 up to 2.5% by weight based on the total weight of the colloidal solution.

A preferred embodiment is a colloidal solution comprising dexpanthenol as active ingredient, a membrane-forming molecule, a water as solvent, ethanol as solubilizer, and a foaming component selected from the group consisting of sodium cocoamphoacetate, capryl/capramidopropyl betaine, the mixture of sodium lauryl glucose carboxylate / lauryl glucoside or a mixture thereof.

Most preferred is a colloidal solution comprising dexpanthenol as active ingredient, phosphatidylcholine as membrane-forming molecule, water as solvent, ethanol as solubilizer and a foaming component selected from the group consisting of sodium cocoamphoacetate, capryl/capramidopropyl betaine, the mixture of sodium lauryl glucose carboxylate / lauryl glucoside or a mixture thereof, wherein the ratio of water and ethanol is for example from 120 : 1 up to 250 : 1.

The colloidal solution of the present invention can be used as foam or spray. Therefore propellants known to the skilled person can be added. Preference is given to compressed nitrogen.

Moreover, the colloidal solution according to the invention can contain further excipients e.g. stabilizers, pH-adjuster, wetting agents, colorants, flavorants, fragrances, viscousifying agents, salts for varying the osmotic pressure, masking agents and antioxidants. It can also contain still other therapeutically valuable substances, such as further vitamins, minerals, sun filters, phytotherapeutic extracts, etc..

Excipients for pharmaceuticals and cosmetically compositions to the skilled person are also described for example in the following handbook: "Handbook of Pharmaceutical Excipients", Wade, A. & Weller, P.J., American Pharmaceutical Association, Washington, 2nd edition 1994.

### Cosmetic or pharmaceutical composition

The colloidal solution according to the invention is a pharmaceutical and/or a cosmetic preparation, in particular a preparation for skin care, which preferably can be used as a foam, foam-spray or spray formulation.

The colloidal solution according to the invention includes but is not limited to a liquid composition before producing a foam, a liquid composition before spraying, the foam itself or the atomized spray itself.

### Use of the composition

The colloidal solution according to the invention can be used for treating dermatological disorders which involve the degranulation process of the mastocytes, such as atopic dermatitis, psoriasis, contact eczema, skin allergies, skin inflammation due to insect bites, skin allergies, senile pruritus, minor bums and sunburns, irritated skin.

Furthermore the colloidal solution according to the invention can be used for moisturization, for regeneration or repair of barrier function of the horny layer, for cooling, and/or for soothing.

Preference is given to a colloidal solution that can be applied dermal or topically one or more times per day.

### Process for manufacture

The colloidal solution according to the invention can be produced by mixing the buffering agent, the preservative and the chelating agent into the solvent, preferably water, at temperature from optionally room temperature up to 50°C, preferably 35 to 40°C. The mixture is stirred until it is dissolved. A part of the active ingredient is then added optionally dissolved in a solvent, preferably in water, more preferably in a concentration of 50 to 80% by weight, most preferably as a 65% by weight aqueous solution. The mixture is stirred and homogenized until dissolution and the temperature can be decreased to 15°C up to room temperature, preferably to 20°C. In the next step a solution comprising at least parts of the active ingredient, at least one membrane-forming molecule, water as solvent, ethanol as solubilizer and a surfactant is added. Preferably a solution comprising dexpanthenol, more preferably in a concentration of 10 to 50%, most preferably in a concentration of 25 to 35 % by weight, water as solvent, ethanol as solubilizer, preferably in a concentration of 6 to 12%, most preferably in a concentration of 8 to 10% by weight, phosphatidylcholine as membrane-forming molecule, preferably in a concentration of 8 to 16%, most preferable in a concentration of 11 to 13% by weight and sodium oleate as surfactant, preferable in a concentration of 0.2 to 0.8%, most preferably in a concentration of 0.4 to 0.6 % by weight is added and the mixture is stirred and homogenized until homogeneously dispersed. Finally the foaming component is added, preferable in a concentration of 0.5 to 5%, most preferably in a concentration of 1 to 3 % by weight.

The mixing is carried out by using a standard homogenization apparatus equipped with stirring and homogenization device.

### Examples:

### Example 1: (Foam Spray)

| **Names of ingredients of the solution** | **Quantity (%,w/w)** | **Function** |
|---|---|---|
| Purified water | 91.02 | Solvent |
| Dexpanthenol | 5.0 | Active ingredient |
| Ethanol | 0.45 | Solubilizer |
| Phosphatidylcholine from soy beans | 0.6 | Membrane-forming |
| Sodium oleate | 0.025 | Surfactant |
| Phenoxyethanol | 0.7 | Preservative |
| Sodium dihydrogen phosphate dihydrate | 0.127 | Buffering agent |
| Disodium edetate | 0.053 | Chelating agent |
| Disodium phosphate dihydrate | 0.024 | Buffering agent |
| Sodium Lauryl Glucose Carboxylate and Lauryl Glucoside | 2.0 | Foaming agent |
| Sorbic acid | (0.01) | |

| **Foaming system:** | **Quantities g. per can** | |
|---|---|---|
| **Solution** | 75.000 | Solution |
| **isobutane/Isopentane (18:82)** | 1.5 to 1.9 | Cooling agent |
| **Compressed Nitrogen** | - | Propellant |

### Example 2: (Foam Spray)

| **Names of ingredients of the solution** | **Quantity (%,w/w)** | **Function** |
|---|---|---|
| Purified water | 91.31 | Solvent |
| Dexpanthenol | 5.0 | Active ingredient |
| Ethanol | 0.45 | Solubilizer |
| Phosphatidylcholine from soy beans | 0.475 | Membrane- forming |
| Sodium oleate | 0.025 | Surfactant |
| Phenoxyethanol | 0.5 | Preservative |
| Sodium dihydrogen phosphate dihydrate | 0.127 | Buffering agent |
| Disodium edetate | 0.053 | Chelating agent |
| Disodium phosphate dihydrate | 0.024 | Buffering agent |
| Capryl/Capramidopropyl Betaine | 2.0 | Foaming agent |

| **Foaming system:** | **Quantities g. per can** | |
|---|---|---|
| **Solution** | 75.000 | Solution |
| **Isobutane/Isopentane (18:82)** | 1.5 to 1.9 | Cooling agent |
| **Compressed Nitrogen** | - | Propellant |

### Example 3: (Foam Spray)

| **Names of ingredients of the solution** | **Quantity (%,w/w)** | **Function** |
|---|---|---|
| Purified water | 91.336 | Solvent |
| Dexpanthenol | 5.0 | Active ingredient |
| Ethanol | 0.45 | Solubilizer |
| Phosphatidylcholine from soy beans | 0.6 | Membrane-forming |
| Sodium oleate | 0.025 | Surfactant |
| Phenoxyethanol | 0.5 | Preservative |
| Sodium dihydrogen phosphate dihydrate | 0.127 | Buffering agent |
| Disodium edetate | 0.053 | Chelating agent |
| Disodium phosphate dihydrate | 0.024 | Buffering agent |
| Sodium Cocoamphoacetate | 2.0 | Foaming agent |
| Citric acid | 0.085 | ph adjuster |

| **Foaming system:** | **Quantities g. per can** | |
|---|---|---|
| **Solution** | 75.000 | Solution |
| **Isobutane/Isopentane (18:82)** | 1.5 to 1.9 | Cooling agent |
| **Compressed Nitrogen** | - | Propellant |

### Stability test:

Examples 1 to 3 and the colloidal solution concentrate are stored at 50°C for 3 month. Analysis of the colloidal solutions show that they remain stable and are not separated or agglomerate to bigger aggregates, i.e. the particle size is less or equal than 100 nm.

### Manufacturing process:

### Examples 1 to 3 are prepared following the same protocol, e.g. for a batch of 2.000 kg:

The buffering agents, the preservative and the chelating agent are added into 1772 kg water. The mixture is stirred at 35-40°C until a solution is formed. 113,5 kg of an about 65% by weight aqueous solution of dexpanthenol is then added. The mixture is homogenized and stirred until dissolution and the temperature is decreased to 20°C. In the next step 100 kg of a colloidal solution concentrate comprising 30 kg dexpanthenol, sodium oleate, phosphatidylcholine, ethanol and water is added and the mixture is homogenized and stirred until homogeneously dispersed. Finally the foaming component is added and the solution is stirred until a homogenously dispersed colloidal solution is achieved.

## Claims

1. Colloidal solution as basis for a foam spray formulation comprising
- at least one active ingredient,
- at least one membrane-forming molecule, and
- at least one foaming component, whereby
the active ingredient is dexpanthenol,
the membrane-forming molecule is phosphatidylcholine, and
the foaming component is selected from the group consisting of sodium oleate, sodium cocoamphoacetate, capryl/capramidopropyl betaine, a mixture of disodium PEG-5 laurylcitrate / sulfosuccinat / sodium laureth sulphate, a mixture of sodium lauryl glucose carboxylate / lauryl glucoside, or a mixture thereof.

2. Colloidal solution of claim 1 wherein the particle size is less equal than 100 nm.

3. Colloidal solution of any of claims 1 to 2, wherein the active ingredient is present in a concentration of from 0.1 up to 20% by weight based on the total weight of the colloidal solution.

4. Colloidal solution of any of claims 1 to 3, wherein the membrane-forming molecule is present in a concentration of from 0.05 up to 15% by weight based on the total weight of the colloidal solution.

5. Colloidal solution of any of claims 1 to 4, wherein the foaming component is present in a concentration of from 0.5 up to 10% by weight based on the total weight of the colloidal solution.

6. Colloidal solution of any of claims 1 to 5 comprising in addition one or more solvent, buffering agent, chelating agent, preservative and/or propellant.

7. Colloidal solution of any of claims 1 to 6 comprising water and ethanol as solvent, wherein water is comprised in a concentration of up to 95% and ethanol in a concentration from 0.1 up to 2% by weight based on the total weight of the colloidal solution.

8. Colloidal solution of any of claims 1 to 7 which is a liquid colloidal solution before producing a foam or before spraying, or the foam or the atomized spray itself.

9. Process for manufacturing of the colloidal solution of any of claims 1 to 8 wherein the active ingredient, the membrane-forming molecule and the foaming component are added to a solvent.

10. Process of claim 9 wherein a solution comprising at least parts of the active ingredient, at least one membrane-forming molecule, water as solvent, ethanol as solubilizer and a surfactant is added to a mixture containing a buffering agent, a preservative, a chelating agent, a solvent and the active ingredient.

11. Colloidal solution of any of claims 1 to 8 for use as a medicament.

12. Colloidal solution of any of claims 1 to 8 for treating atopic dermatitis, psoriasis, contact eczema, skin allergies, skin inflammation due to insect bites, skin allergies, senile pruritus, minor bums and sunburns, or irritated skin.

## Patentansprüche

1. Kolloidale Lösung als Grundlage für eine Schaumsprayformulierung, umfassend
- mindestens einen Wirkstoff,
- mindestens ein membranbildendes Molekül und
- mindestens eine schäumende Komponente, wobei es sich bei dem Wirkstoff um Dexpanthenol handelt, es sich bei dem membranbildenden Molekül um Phosphatidylcholin handelt und
die schäumende Komponente aus der Gruppe bestehend aus Natriumoleat, Natriumcocoamphoacetat, Capryl/Capramidopropylbetain, einer Mischung von Dinatrium-PEG-5-laurylcitrat, Sulfosuccinat und Natriumlaurethsulfat, einer Mischung von Natrium-laurylglucosecarboxylat und Laurylglucosid oder einer Mischung davon ausgewählt ist.

2. Kolloidale Lösung nach Anspruch 1, wobei die Teilchengröße kleiner gleich 100 nm ist.

3. Kolloidale Lösung nach einem der Ansprüche 1 bis 2, wobei der Wirkstoff in einer Konzentration von 0,1 bis 20 Gew.-%, bezogen auf das Gesamtgewicht der kolloidalen Lösung, vorliegt.

4. Kolloidale Lösung nach einem der Ansprüche 1 bis 3, wobei das membranbildende Molekül in einer Konzentration von 0,05 bis 15 Gew.-%, bezogen auf das Gesamtgewicht der kolloidalen Lösung, vorliegt.

5. Kolloidale Lösung nach einem der Ansprüche 1 bis 4, wobei die schäumende Komponente in einer Konzentration von 0,5 bis 10 Gew.-%, bezogen auf das Gesamtgewicht der kolloidalen Lösung, vorliegt.

6. Kolloidale Lösung nach einem der Ansprüche 1 bis 5, die außerdem ein oder mehrere Lösungsmittel, eine oder mehrere Puffersubstanzen, einen oder mehrere Chelatbildner, einen oder mehrere Konservierungsstoffe und/oder ein oder mehrere Treibmittel umfasst.

7. Kolloidale Lösung nach einem der Ansprüche 1 bis 6, die Wasser und Ethanol als Lösungsmittel umfasst, wobei Wasser in einer Konzentration von bis zu 95 Gew.-% und Ethanol in einer Konzentration von 0,1 bis 2 Gew.-%, bezogen auf das Gesamtgewicht der kolloidalen Lösung, enthalten ist.

8. Kolloidale Lösung nach einem der Ansprüche 1 bis 7, bei der es sich um eine flüssige kolloidale Lösung vor der Herstellung eines Schaums oder vor dem Sprühen oder den Schaum oder das zerstäubte Spray selbst handelt.

9. Verfahren zur Herstellung der kolloidalen Lösung nach einem der Ansprüche 1 bis 8, bei dem der Wirkstoff, das membranbildende Molekül und die schäumende Komponente zu einem Lösungsmittel gegeben werden.

10. Verfahren nach Anspruch 9, bei dem eine Lösung, die mindestens Teile des Wirkstoffs, mindestens ein membranbildendes Molekül, Wasser als Lösungsmittel, Ethanol als Lösungsvermittler und ein Tensid umfasst, zu einer Mischung, die eine Puffersubstanz, einen Konservierungsstoff, einen Chelatbildner, ein Lösungsmittel und den Wirkstoff enthält, gegeben wird.

11. Kolloidale Lösung nach einem der Ansprüche 1 bis 8 zur Verwendung als Medikament.

12. Kolloidale Lösung nach einem der Ansprüche 1 bis 8 zur Behandlung von atopischer Dermatitis, Psoriasis, Kontaktekzem, Hautallergien, Hautentzündung infolge von Insektenstichen, Hautallergien, Pruritus senilis, kleineren Verbrennungen und Sonnenbränden oder gereizter Haut.

## Revendications

1. Solution colloïdale comme base d'une formule de pulvérisation de mousse comprenant
- au moins un principe actif,
- au moins une molécule formant une membrane, et
- au moins un composant moussant, où
le principe actif est le dexpanthénol,
la molécule formant une membrane étant la phosphatidylcholine, et
le composant moussant est choisi dans le groupe constitué par les suivants : oléate de sodium, cocoamphoacétate de sodium, capryl/capramidopropylbétaïne, un mélange de laurylcitrate de disodium PEG-5/sulfosuccinal/laurethsulfate de sodium, un mélange de laurylglucosecarboxylate de sodium/laurylglucoside, ou l'un de leurs mélanges.

2. Solution colloïdale selon la revendication 1, où la granulométrie est inférieure ou égale à 100 nm.

3. Solution colloïdale selon l'une quelconque des revendications 1 à 2, où le principe actif est présent à une teneur comprise entre 0,1 et 20 % en masse par rapport à la masse totale de la solution colloïdale.

4. Solution colloïdale selon l'une quelconque des revendications 1 à 3, où la molécule formant une membrane est présente à une concentration comprise entre 0,05 et 15 % en masse par rapport à la masse totale de la solution colloïdale.

5. Solution colloïdale selon l'une quelconque des revendications 1 à 4, où le composant moussant est présent à une concentration comprise entre 0,5 et 10 % en masse par rapport à la masse totale de la solution colloïdale.

6. Solution colloïdale selon l'une quelconque des revendications 1 à 5 comprenant de plus un ou plusieurs solvants, agents tampons, agents chélatants, conservateurs et/ou propulseurs.

7. Solution colloïdale selon l'une quelconque des revendications 1 à 6, comprenant de l'eau et de l'éthanol en tant que solvant, où l'eau est incluse à une concentration allant jusqu'à 95 % et de l'éthanol à une concentration comprise entre 0,1 et 2 % en masse par rapport à la masse totale de la solution colloïdale.

8. Solution colloïdale selon l'une quelconque des revendications 1 à 7, qui est une solution colloïdale liquide avant production d'une mousse ou avant pulvérisation, ou la mousse ou la pulvérisation atomisée elle-même.

9. Procédé de fabrication de la solution colloïdale selon l'une quelconque des revendications 1 à 8, où le principe actif, la molécule formant une membrane et le composant moussant sont ajoutés à un solvant.

10. Procédé selon la revendication 9, où une solution comprenant au moins des parties du principe actif, au moins une molécule formant une membrane, de l'eau en tant que solvant, de l'éthanol en tant que solubilisant et un tensioactif, est ajoutée à un mélange contenant un agent tampon, un conservateur, un agent chélatant, un solvant et le principe actif.

11. Solution colloïdale selon l'une quelconque des revendications 1 à 8, pour utilisation en tant que médicament.

12. Solution colloïdale selon l'une quelconque des revendications 1 à 8 pour le traitement de la dermatite atopique, du psoriasis, de l'eczéma de contact, des allergies cutanées, de l'inflammation cutanée due aux morsures d'insectes, des allergies cutanées, du prurit sénile, des brûlures mineures ou des coups de soleil, ou des irritations cutanées.
